# EUROPEAN PATENT SPECIFICATION

(11) **EP 2 042 197 B1**
(45) Date of publication and mention of the grant of the patent: **01.08.2012**
(21) Application number: 07745054.2
(22) Date of filing: 12.06.2007
(51) Int. Cl.: A61L 9/00, A61L 9/16, A61L 9/22, B03C 3/02, B03C 3/41, A61L 9/014, F24F 3/16

(54) **AIR CLEANER**
LUFTREINIGUNGSVORRICHTUNG
ÉPURATEUR D'AIR

(30) Priority: 05.07.2006 JP 2006185976
(43) Date of publication of application: 01.04.2009
(73) Proprietor: Daikin Industries, Ltd., Osaka 530-8323 (JP)
(72) Inventor: YAMASHITA, Tetsuya, Kusatsu-shi Shiga 525-8526 (JP); NAGAO, Mitsuhisa, Kusatsu-shi Shiga 525-8526 (JP)
(74) Representative: Goddar, Heinz J.
(86) International application number: PCT/JP2007/061767
(87) International publication number: WO 2008/004411

(56) References cited:
- EP-A- 0 431 648
- WO-A-2007/083573
- JP-A- 05 208 116
- JP-A- 2003 236 332
- JP-A- 2004 305 395
- JP-A- 2005 300 111
- JP-A- 2006 503 609

## Description

### TECHNICAL FIELD

The present invention relates to an air cleaner.

### BACKGROUND ART

Conventionally, there are air cleaners that remove odorous components, dust, fungi and viruses floating in air inside a room and again return the air that has been thereby cleaned to the inside of the room. Such air cleaners perform cleaning by decomposing, killing or inactivating the odorous components, dust and viruses by ozone.

With respect thereto, in recent years, for example, such as in an air cleaner described in Patent Document 1 indicated below, there has been proposed an air cleaner that is disposed with plural stages of cleaning, such as a stage where the air cleaner removes dust that has been made electrostatic using an electrostatic dust collecting filter and a stage where the air cleaner causes dust, odorous components and viruses floating in the air to be adsorbed by an adsorption portion, generates active species of high-speed electrons, ions and hydroxy radicals whose reaction activity is regarded as high and sends these to the adsorption portion to perform cleaning.
<Patent Document 1> JP-A No. 2005-300111

Other air cleaners are known from eg WO 2007/083573 A, EP 1 980 317, JP 2004 305395 A, EP 0 431 648 A and JP 2003 236332 A.

### DISCLOSURE OF THE INVENTION

### PROBLEM THAT THE INVENTION IS TO SOLVE

However, in the air cleaner described in the aforementioned Patent Document 1, the air that passes through the portion that generates the active species still includes contaminants in the air. For this reason, there is the potential for contaminants such as dust to adhere to the surface of a plasma discharge electrode that generates the active species as a result of being exposed to such contaminated air and for the contaminants to end up accumulating and inhibit the generation of plasma discharge.

It will be noted that, although the adhesion of contaminants can be avoided by disposing the position of the plasma discharge electrode on the downstream-most side of the plural cleaning stages, in this case, the air cleaner cannot perform, on the upstream side, cleaning utilizing the air that has been plasma-discharged.

The present invention has been made in view of the aforementioned points, and it is an object thereof to provide an air cleaner which, even when the air cleaner employs a cleaning function for which there is the potential for the exhibition efficiency of the function to be reduced by contaminants in the air, is capable of stabilizing the exhibition of that function.

### MEANS FOR SOLVING THE PROBLEM

An air cleaner pertaining to a first aspect of the present invention is an air cleaner that allows air of a target space to pass therethrough and cleans the air, the air cleaner comprising a first flow path that interconnects a suction opening for sucking in the air of the target space and a blowout opening for blowing out the air with respect to the target space, a first cleaning portion (40) disposed in the first flow path and having a filter means (41a) that passes therethrough from an upstream side to a downstream side; a second flow path that interconnects a downstream side connecting space between the first cleaning means and the blowout opening in the first flow path and a upstream side connecting space between the first cleaning means and the suction opening in the first flow path, and a second cleaning means (63) disposed in the second flow path and having an electrode (70) and a ground plate (73), an additional function of causing the air that passes therethrough from the downstream side of the first cleaning portion to the upstream side of the first cleaning portion in a state that additionally allows a second cleaning function that is different from the first cleaning_function to be exhibited in the first cleaning portion (40), wherein a blower (31) disposed in a position in the first flow path between the first cleaning means and the downstream side connecting space, and at which position the blower (31) makes both the vicinity of the blowout opening and the vicinity of one end of the second flow path facing to the downstream side connecting space into a positive pressure as a result of the blower (31) being driven to rotate; and the blower (31) forms an air flow that leads from the suction opening to the blowout opening in the first flow path and an air flow that leads from the downstream side connecting space to the upstream side connecting space in the second flow path.

### EFFECTS OF THE INVENTION

when the air cleaner employs a cleaning function for which there is the potential for the exhibition efficiency of the function to be reduced by contaminants in the air, it becomes possible for the air cleaner to stabilize the exhibition of that function.

it becomes possible to effectively prevent deterioration of the second cleaning portion by contaminants in the air.

it becomes possible to make the exhibition of the second function in the first cleaning portion effective.

it becomes possible to effectively remove the cleaning target that is adsorbed by the adsorbing means.

it becomes possible for the cleaning target that has been efficiently trapped to be effectively cleaned by the second cleaning function.

it becomes possible for relatively large particles of the cleaning target that have been trapped to be effectively cleaned by the second cleaning function.

even when the air cleaner employs a cleaning function for which there is the potential for the exhibition efficiency of the function to be reduced by contaminants in the air, it becomes possible for the air cleaner to stabilize the exhibition of that function.

### BRIEF DESCRIPTION OF THE DRAWINGS

FIG. 1 is an external perspective diagram of an air cleaner pertaining to an embodiment of the invention.
FIG. 2 is an external perspective diagram of the air cleaner in a state where a front panel has been removed.
FIG 3 is an external perspective diagram of the back side of the air cleaner.
FIG 4 is a diagram showing the position of a fan in the air cleaner.
FIG. 5 is an explanatory diagram of air cleaning by a main air flow and a bypass air flow.
FIG. 6(a) is a front diagram showing a main air flow path and a bypass air flow path.
FIG 6(b) is a side diagram showing the main air flow path and the bypass air flow path.
FIG. 6(c) is a top diagram showing the main air flow path and the bypass air flow path.
FIG. 7 is a configuration diagram of a unit housing portion.
FIG. 8 is an external perspective diagram of a streamer discharge unit.
FIG. 9 is a cross-sectional diagram of the streamer discharge unit as seen from above.
FIG 10 is a rear perspective diagram of a plasma ionization portion.
FIG. 11 is a top diagram of the plasma ionization portion.
FIG. 12 is a front diagram when a prefilter is attached to the plasma ionization portion.
FIG 13 is an enlarged diagram of relevant portions of the prefilter.
FIG. 14 is an enlarged diagram of relevant portions of the plasma ionization portion.
FIG. 15 is a diagram showing an extension passage pertaining to a modification (C).

### DESCRIPTION OF THE REFERENCE NUMERALS

1: Air Cleaner
12: Blowout Opening
13: Lower Suction Opening
14: Side Suction Opening
31: Blowing Fan
40: Air Cleaning Unit (First Cleaning Portion)
41: Prefilter
41a: Filter Portion (First Passage Portion)
41b: Partition Ventilation Portion (Filter Partition Portion)
410: Hole (Second Passage Portion)
42: Plasma Ionization Portion (Downstream Member)
43: Photocatalytic Filter
44: Plasma Catalytic Filter
63: Streamer Discharge Unit

### BEST MODE FOR CARRYING OUT THE INVENTION

Below, an embodiment of an air cleaner pertaining to the present invention will be described on the basis of the drawings.

### <Configuration of Air Cleaner 1>

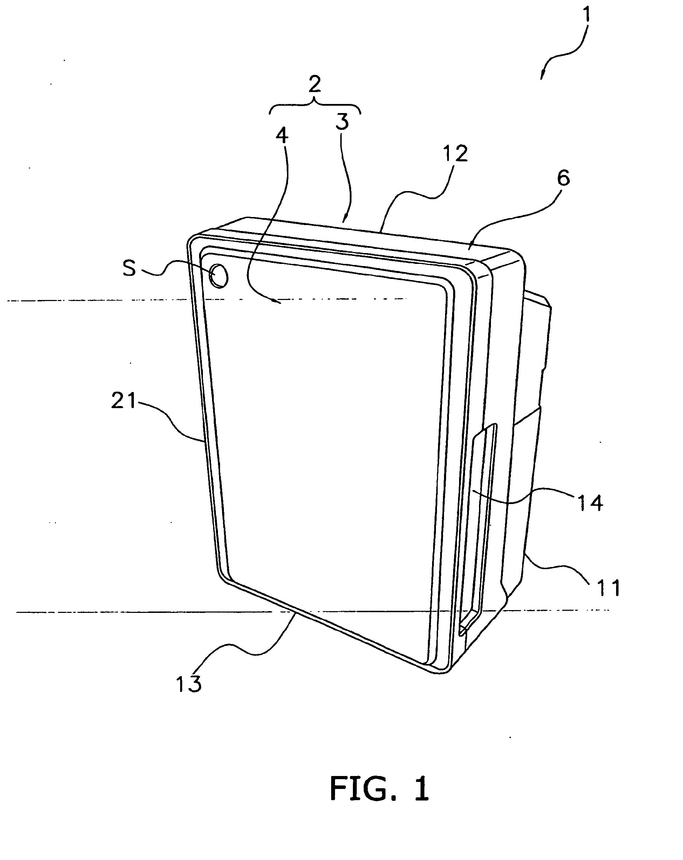

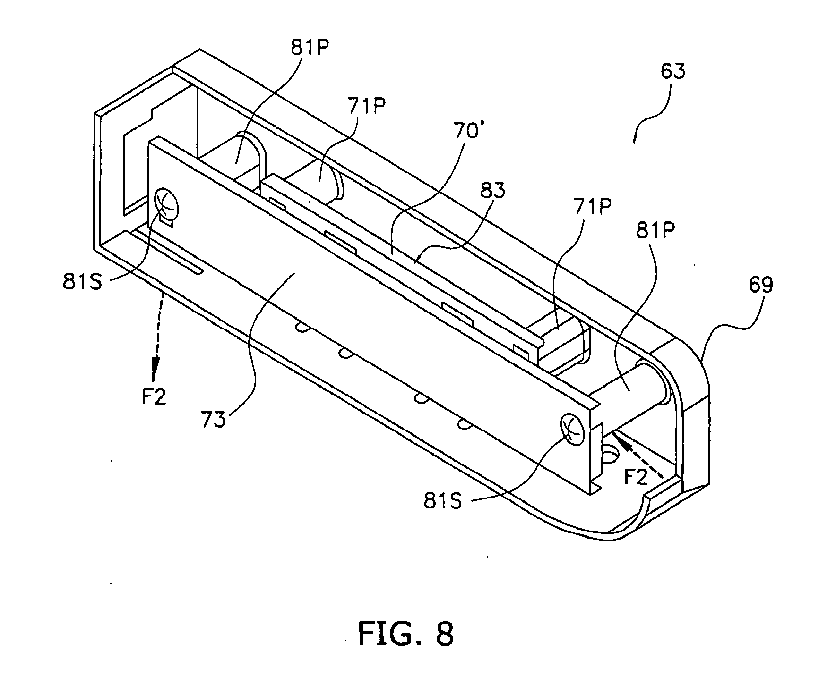

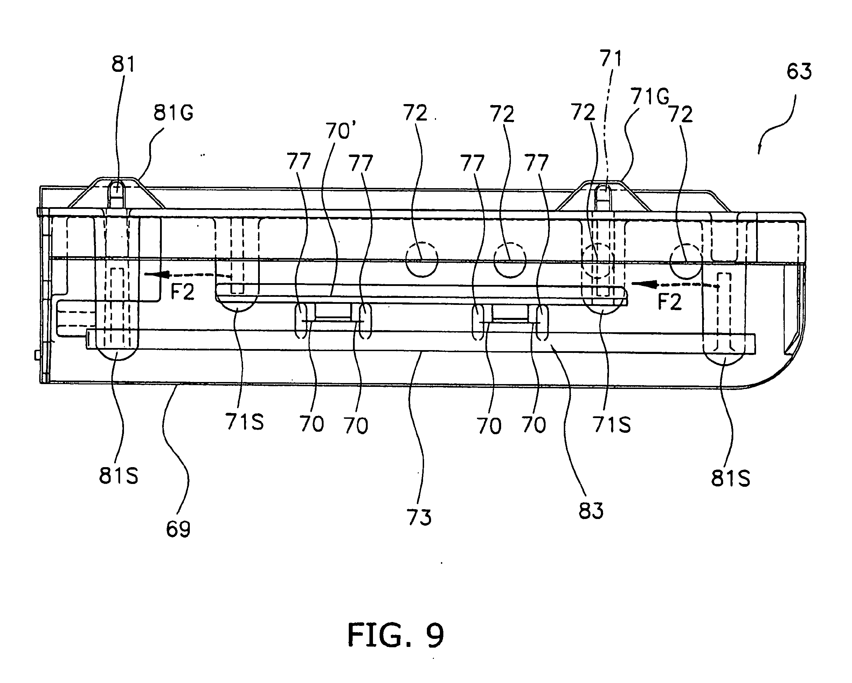

FIG. 1 shows an air cleaner 1 pertaining to an embodiment of the present invention. This air cleaner 1 is a floor-standing air cleaner that is installed on a floor inside a room in order to maintain the air inside the room clean and improve comfort inside the room. The air cleaner 1 is equipped with a body portion 2 and a streamer discharge unit 63 (see FIG. 8 and FIG. 9). The body portion 2 is configured to be dividable front and back, with a first body portion 3 being disposed on the back side and a second body portion 4 being disposed on the front side. The streamer discharge unit 63 is positioned so as to extend in a front-rear direction on the upper right side of the body portion 2 when seen from the front.

### <First Body Portion 3>

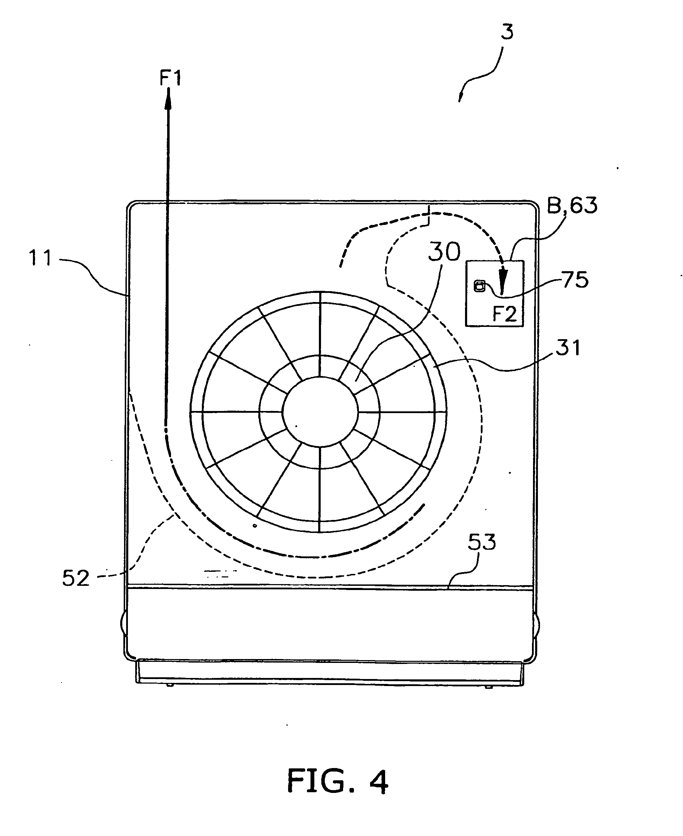

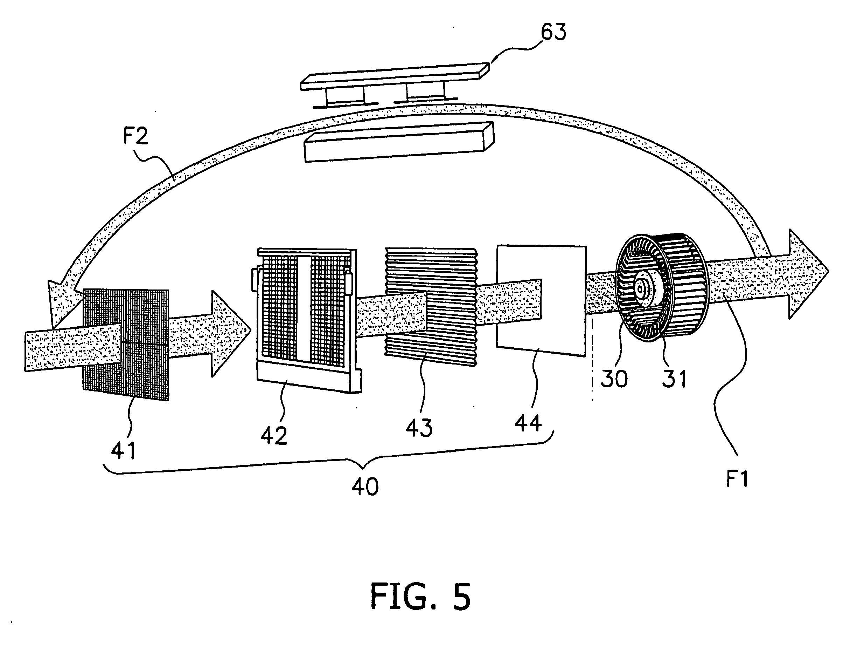

The first body portion 3 includes, as shown in FIG. 4, a first body casing 11, a fan motor 30, a blowing fan 31 (blowing device), a unit housing portion B, a photocatalytic filter 43 (see FIG. 5) and a plasma catalytic filter 44 (see FIG 5). It will be noted that FIG 4 is a front diagram of the first body portion 3 in a state where the photocatalytic filter 43 and the plasma catalytic filter 44 have been removed.

### {First Body Casing 11}

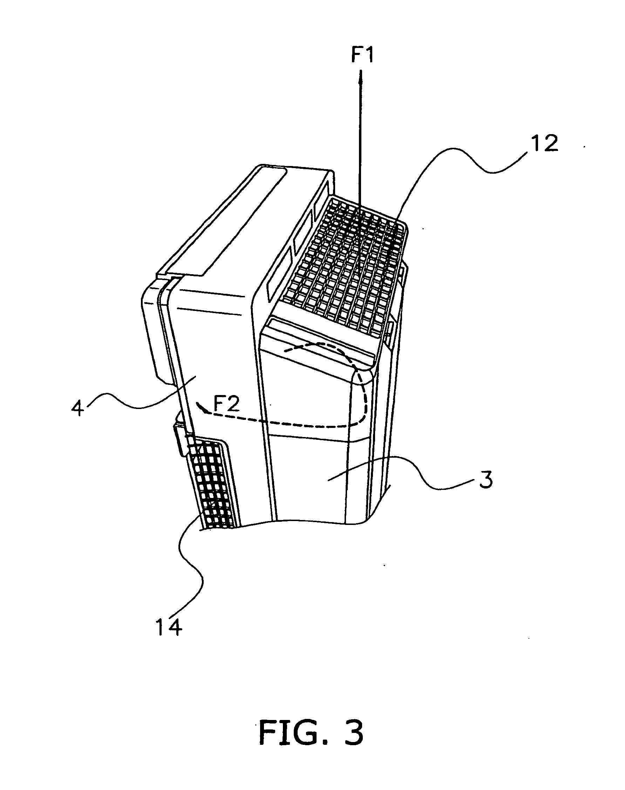

The first body casing 11 internally houses the fan motor 30, the blowing fan 31, the unit housing portion B, the photocatalytic filter 43, the plasma catalytic filter 44 and the like; as shown in FIG. 1 and FIG. 3, the first body casing 11 includes a blowout opening 12, a lower suction opening 13 and side suction openings 14. The blowout opening 12 is disposed on the rear side end portion of the top surface portion of the first body casing 11. The blowout opening 12 is an opening for blowing out air that has been cleaned upward from the air cleaner 1. The lower suction opening 13 and the side suction openings 14 are substantially rectangular openings for sucking the air inside the room into the inside of the air cleaner 1. The lower suction opening 13 is disposed in the lower front side end portion of the first body casing 11 that is the same as the side where the blowout opening 12 is disposed. The transverse direction length of the lower suction opening 13 is substantially the same as the transverse direction length of a front panel 21. The side suction openings 14 are a pair of openings that are respectively disposed on the front side of the right and left side surface portions of the first body casing 11.

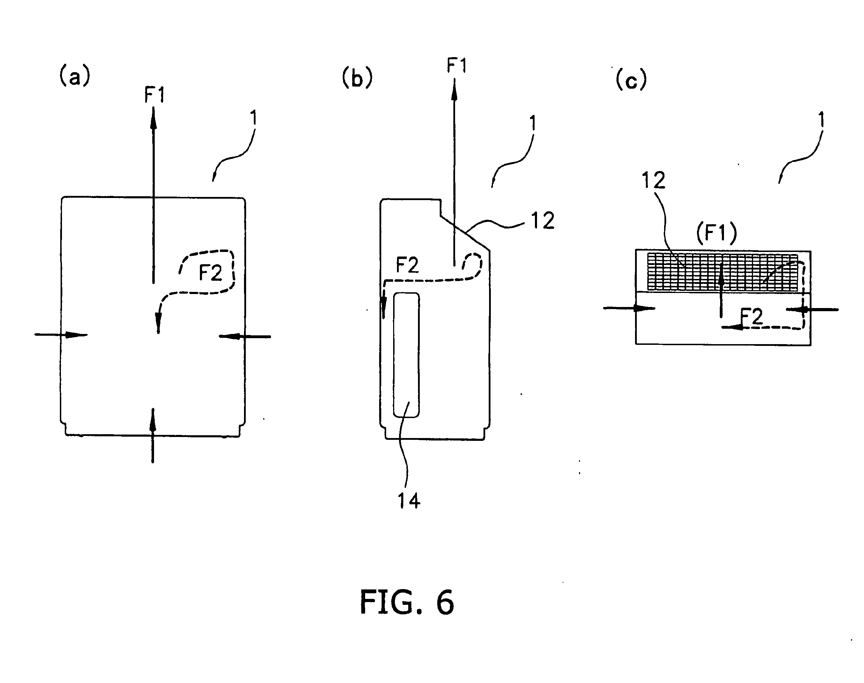

It will be noted that the first body casing 11 configures a body casing 6 together with the later-described front panel 21. Inside the body casing 6, there are disposed a main air flow path, through which passes air that is sucked in through the suction openings 13, 14 and 22 from the inside of the room, is cleaned in an air cleaning unit 40 and is blown out into the inside of the room from the blowout opening 12, and a bypass air flow path, which returns the air in the vicinity of the blowout opening 12 to the upstream side; as shown in FIG 3, FIG. 4 and FIGS. 6(a), (b) and (c), a main air flow F1 that flows through this main air flow path and a bypass air flow F2 that flows through the bypass air flow path are generated by the blowing fan 31. Here, FIG 6(a) is a schematic diagram of the front of the air cleaner 1, FIG 6(b) is a schematic diagram of the right side of the air cleaner 1, and FIG. 6(c) is a schematic diagram of the top of the air cleaner 1.

It will be noted that, on a more upstream side than the blowing fan 31, the main air flow path generally flows from front to back; below, "front" will mean "the upstream side in the flow direction of the main air flow of the main air flow path" and "back" will mean "the downstream side in the flow direction of the main air flow of the main air flow path". That is, the bypass air flow that flows through the bypass air flow path generally flows from back to front.

Here, as shown in FIG. 3, FIG 4 and FIGS. 6(a), (b) and (c), the main air flow F1 that flows through the main air flow path is an air flow that cleans the air that has been sucked in from the lower suction opening 13 and the side suction openings 14 and blows out the air from the blowout opening 12. Further, the bypass air flow F2 that flows through the bypass air flow path flows upward in the vicinity of the blowout opening 12, flows from back to front in a state where the streamer discharge unit 63 is housed in the unit housing portion B, flows as far as the vicinity of the substantial center of the front of a later-described plasma ionization portion 42, descends, and is guided to the front of a later-described prefilter 41.

### {Blowing Fan 31 and Fan Motor 30}

The blowing fan 31 and the fan motor 30 shown in FIG. 4 and FIG. 5 generate the main air flow F1 that flows through the main air flow path and the bypass air flow F2 that flows through the bypass air flow path. As the blowing fan 31, a centrifugal fan is employed. For this reason, the blowing fan 31 sucks in the air from its axis-of rotation direction and blows out the air outward in its radial direction from its rotational center. The fan motor 30 drives the blowing fan 31 to rotate. As the fan motor 30, an inverter motor whose frequency is controlled by an inverter circuit is employed.

In a first space on the upstream side of the blowing fan 31, there is housed a later-described air cleaning unit 40 (see FIG 5). In a second space on the downstream side of the blowing fan 31, there are housed the fan motor 30, the blowing fan 31 and a scroll 52 that is formed on the side of the blowing fan 31, that is, along a circumferential direction about the axis of rotation of the blowing fan. Additionally, the air that is blown out from the blowing fan 31 is delivered to the inside of the room from the blowout opening 12 along the scroll 52.

### {Photocatalytic Filter 43}

The photocatalytic filter 43 is, as shown in FIG. 5, formed in a pleated shape and is formed by adhering together an electrostatic filter and a titanium apatite-carrying filter. It will be noted that this photocatalytic filter 43 is disposed such that the electrostatic filter faces the front side and such that the titanium apatite-carrying filter faces the back side. The electrostatic filter adsorbs dust and the like that has been charged when the air passes through the later-described plasma ionization portion 42. The titanium apatite-carrying filter adsorbs dust and the like that has passed through the electrostatic filter. This titanium apatite-carrying filter is, similar to the prefilter 41, formed from PP fiber that carries titanium apatite. It will be noted that titanium apatite is apatite where some calcium atoms of calcium hydroxy apatite are substituted with titanium atoms by a technique such as ion exchange. Titanium apatite has the property that it uniquely adsorbs viruses, mold fungi, bacteria and the like that are included in dust and the like. Additionally, the photocatalytic function of titanium apatite is activated by active species that are supplied from the later-described streamer discharge unit 63, and the titanium apatite inactivates or kills the viruses, mold fungi, bacteria and the like.

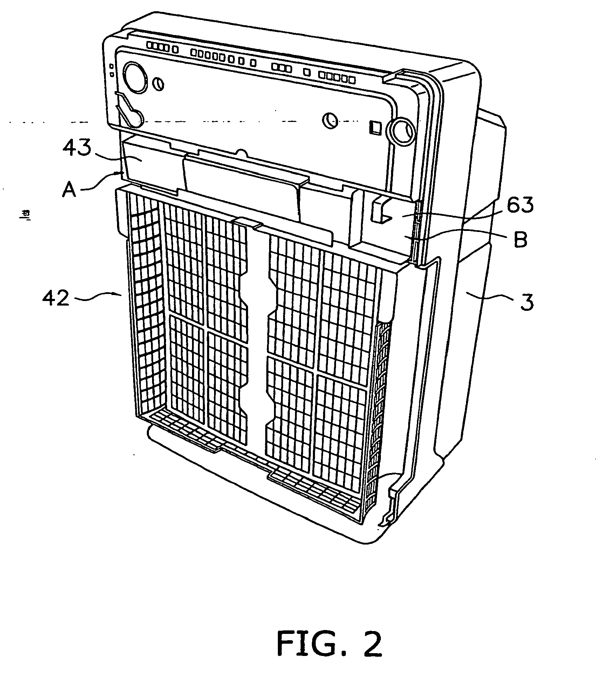

Further, this photocatalytic filter 43 is capable of being easily folded along its creases because it is formed in a pleated shape. For this reason, as shown in FIG. 2, the photocatalytic filter 43 is housed in an exchange-use filter housing portion A that is formed above in a state where the photocatalytic filter 43 is folded.

### {Plasma Catalytic Filter 44}

The plasma catalytic filter 44 is disposed in front of the blowing fan 31 and in back of the photocatalytic filter 43. That is, the plasma catalytic filter 44 is disposed between the blowing fan 31 and the photocatalytic filter 43. In the plasma catalytic filter 44, anatase-form titanium dioxide is carried. The plasma catalytic filter 44 adsorbs viruses, fungi and the like in the air that were not adsorbed by the photocatalytic filter 43. In this plasma catalytic filter 44, fungi, viruses and the like that have been adsorbed are killed or inactivated by the titanium dioxide that has been activated by the active species.

### {Unit Housing Portion B}

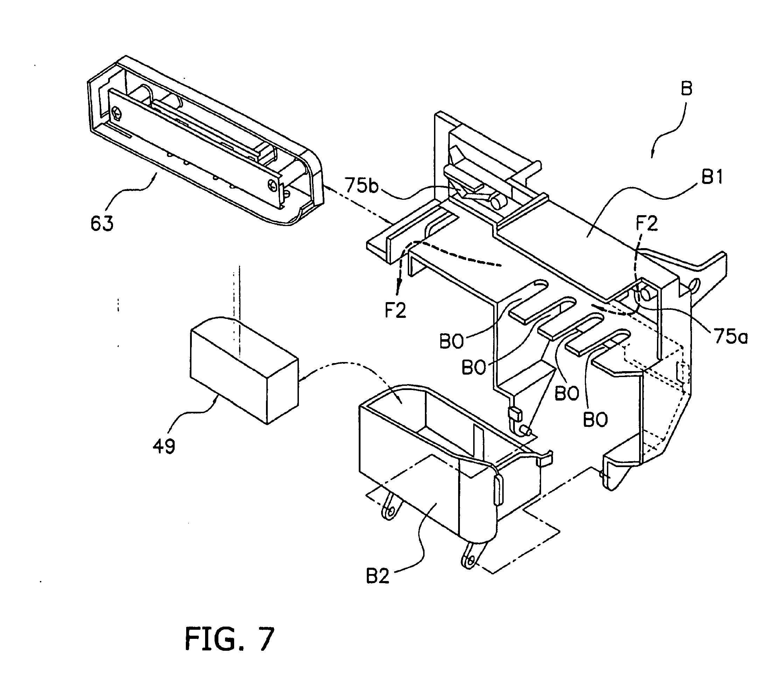

As shown in FIG 7, the unit housing portion B freely removably houses the streamer discharge unit 63 and configures part of the bypass air flow path through which the bypass air flow F2 flows. This unit housing portion B is configured by a unit housing body B1 and a cartridge housing portion B2 that is attached, such that it may be freely opened and closed, with respect to the unit housing body B1.

In this cartridge housing portion B2, there is housed a deodorization catalyst cartridge 49. As shown in FIG. 7, this cartridge housing portion B2 is configured such that the deodorization catalyst cartridge 49 is capable of being replaced in a state where an opening/closing portion has been opened. Deodorization-use openings BO are disposed such that, in a state where the deodorization catalyst cartridge 49 is housed in the cartridge housing portion B2 and the cartridge housing portion B2 is closed, the coming and going of air with the bypass air flow path of the unit housing body B 1 becomes possible. Thus, odors are killed by targeting the air that flows through the bypass air flow path and adsorbing and decomposing odorous components.

In the unit housing body B1, there are disposed body contacts 75a and 75b that become electrically connected and electrically conductive in a state where the streamer discharge unit 63 has been attached. The body contacts 75a and 75b are formed from metal plates and are connected to a power supply via a power supply circuit (not shown) that is housed in the first body portion 3 and a power supply cord (not shown). Thus, the body contacts 75a and 75b are disposed apart from each other in the longitudinal direction and in the vertical direction and transmit electrical current from the power supply to the streamer discharge unit 63 by contacting a later-described discharge unit contact 71. Here, the body contact 75a is positioned on the rear side and contacts streamer discharge electrodes 70, and the body contact 75b is positioned in front and contacts a ground plate 73. It will be noted that the unit housing body B 1 is formed by an insulating material such as a resin.

### <Streamer Discharge Unit 63>

The streamer discharge unit 63 is, as mentioned above, disposed by being housed in the unit housing portion B to the upper right of the first body portion 3 and forms part of the main air flow path (see FIG. 2 and FIG 4).

The streamer discharge unit 63 includes, as shown in FIG. 8 and FIG 9, a discharge unit casing 69, a discharge portion 83 and discharge unit contacts 71 and 81; the streamer discharge unit 63 creates a streamer discharge, whereby the streamer discharge unit 63 generates the active species that are supplied to the photocatalytic filter 43 and releases the active species into the bypass air flow F2.

### {Discharge Unit Casing 69}

The discharge portion 83 is attached to the discharge unit casing 69. The discharge unit casing 69 is a box-shaped member that is formed from a resin and has an outer shape that conforms to that of the unit housing portion B. The top surface and the right side surface of the discharge unit casing 69 are open, and the discharge unit casing 69 covers the front, back, left side surface and bottom surface of the discharge portion 83.

The discharge unit casing 69 is, as shown in FIG 7, inserted into the inside of the unit housing portion B and is freely detachably attached to the unit housing portion B. The discharge unit casing 69 is attached to the unit housing portion B, whereby the discharge unit casting 69 covers the periphery of the discharge portion 83 together with the unit housing portion B and forms the bypass air flow path.

On the left side (upper side shown in FIG. 9) of the rear surface of the discharge unit casing 69, contact covers 71 G and 81 G are disposed in two places apart from each other in the longitudinal direction and in the vertical direction. The contact covers 71 G and 8 1 G have the function of guiding insertion in the front-rear direction by sliding with members of the unit housing portion B when the streamer discharge unit 63 is inserted into the unit housing portion B.

The discharge unit contacts 71 and 81 are, as shown in FIG. 9, disposed apart from each other in the longitudinal direction and in the vertical direction in positions that respectively correspond to the positions of the contact covers 71G and 81 G. In a state where the streamer discharge unit 63 has been attached, the left side shown in FIG. 9 becomes the front of the air cleaner 1 and the right side becomes the back. The discharge unit contact 71 and the discharge unit contact 81 are, as mentioned above, guided by the contact covers 71 G and 81 G and respectively contact the body contact 75a and the body contact 75b in a state where the streamer discharge unit 63 has been inserted into the unit housing portion B.

Further, plural minute holes 77 are disposed in the longitudinal direction intermediate portion of the bottom surface of the discharge unit casing 69.

### {Discharge Portion 83}

The discharge portion 83 is a main portion that creates a streamer discharge and includes the streamer discharge electrodes 70 and the ground plate 73.

### (Streamer Discharge Electrodes 70)

The streamer discharge electrodes 70 are configured by terminals that are attached to portions where a metal plate 70' has been cut and raised; a discharge voltage is applied to the streamer discharge electrodes 70, whereby the streamer discharge electrodes 70 create a streamer discharge between themselves and the ground plate 73. The streamer discharge electrodes 70 and the metal plate 70' are, via two insulating struts 7 1 P and with respect to the discharge unit casing 69, screwed and attached by screws 71S (two) that pass through the insides of the insulating struts 71 P. One of the two insulating struts 71 P is screwed and attached so as to contact the discharge unit contact 71. Thus, in a state where the streamer discharge unit 63 has been attached to the unit housing portion B and the discharge unit contact 71 contacts the body contact 75a, the discharge unit contact 71 becomes electrically connected to the streamer discharge electrodes 70 via the screw 71 S. Thus, the discharge unit contact 71 can transmit the discharge voltage to the streamer discharge electrodes 70 from the body contact 75a.

### (Ground Plate 73)

The ground plate 73 is formed from a metal plate and has a substantially rectangular outer shape that is larger than the metal plate 70' of the streamer discharge electrodes 70. The ground plate 73 is disposed in parallel with respect to the streamer discharge electrodes 70 and is disposed close to but apart from the streamer discharge electrodes 70. The ground plate 73 is, via two insulating struts 81 P and with respect to the discharge unit casing 69, screwed and attached by screws 81S (two) that pass through the insides of the insulating struts 81P. One of the two insulating struts 81P is screwed and attached so as to contact the discharge unit contact 81. Thus, in a state where the streamer discharge unit 63 has been attached to the unit housing portion B and the discharge unit contact 81 contacts the body contact 75b, the discharge unit contact 81 becomes electrically connected to the ground plate 73 via the screw 81S.

As mentioned above, the body contact 75a that corresponds to the streamer discharge electrodes 70 is positioned in back of the body, so it is difficult for the hands of a user to reach it, and safety is ensured. Further, the streamer discharge electrodes 70 and the ground plate 73 are respectively fixed to the discharge unit casing 69 that is formed by a resin via the insulating struts 71P and 81P, so insulation is ensured.

It will be noted that, in the above configuration, the discharge unit casing 69 partitions between a space where the streamer discharge electrodes 70 and the ground plate 73 are disposed and a space where contact points of the discharge unit contacts 71 and 81 are disposed. Thus, the contact points of the discharge unit contacts 71 and 81 are disposed in a space that is partitioned from the space where the streamer discharge electrodes 70 and the ground plate 73 are disposed.

In this manner, the active species that are generated from the streamer discharge electrodes 70 are released with respect to the bypass air flow that flows through the bypass air flow path. The active species that have been released in this streamer discharge unit 63 flow along a top surface guide 42G and a front surface guide 42H of the later-described plasma ionization portion 42 and are supplied to the front of the prefilter 41.

### <Second Body Portion 4>

The second body portion 4 is, as shown in FIG. 1, FIG. 2 and FIG. 5, freely detachably attached to the front of the first body portion 3. The second body portion 4 includes the front panel 21, the prefilter 41 (see FIG. 5) and the plasma ionization portion 42 (see FIG 5).

### {Front Panel 21}

The front panel 21 is disposed on the front of the second body portion 4. The height of this front panel 21 is longer than the depth of the air cleaner 1. Further, here, a semitransparent portion S is disposed such that sensing by a human detection sensor (not shown) that is used in order to sense human movement and control ON/OFF and the like becomes possible.

### {Prefilter 41}

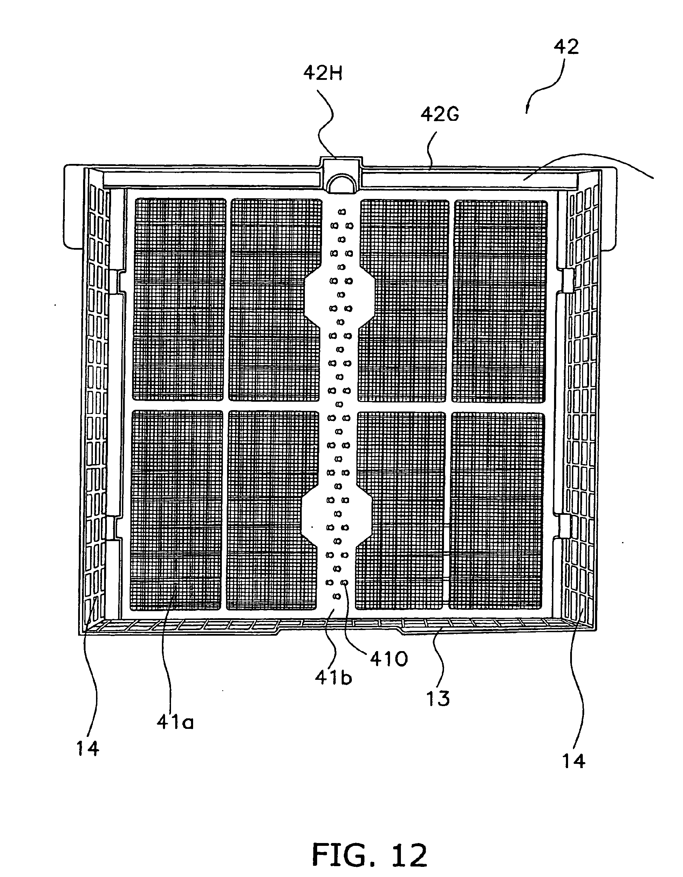

As shown in FIG. 5 and FIG. 12, the prefilter 41 is disposed with filter portions 4 1 a and a partition ventilation portion 41 b.

The filter portions 41 are disposed in back of the front panel 21 and remove relatively large particles of dust. The filter portions 41 a are configured from a net that comprises a filiform resin net that is made of polypropylene (below, called PP) and a frame that holds the net. A visible light-type photocatalyst and a catechin are carried, so as to be exposed to the air, in the fiber that configures the net of the filter portions 41a. The visible light-type photocatalyst includes titanium oxide and the like whose photocatalytic action is activated by visible light and removes viruses and fungi such as mold fungi and bacteria that are included in dust and the like that adheres to the filter portions 41a. It will be noted that the catechin is a type of polyphenol and is a collective name for epicatechin, epigallocatechin, epicatechin gallate, epigallocatechin gallate and the like. This catechin suppresses the breeding of fungi such as mold fungi and bacteria that are included in dust and the like that adheres to the filter portions 41 a and inactivates viruses.

The partition ventilation portion 41 b is, as shown in FIG. 12, disposed vertically in the center of the prefilter 41 so as to partition the right and left filter portions 41 a. This partition ventilation portion 41 b includes plural holes 410 that penetrate the partition ventilation portion 41 b in the front-rear direction.

### {Plasma Ionization Portion 42}

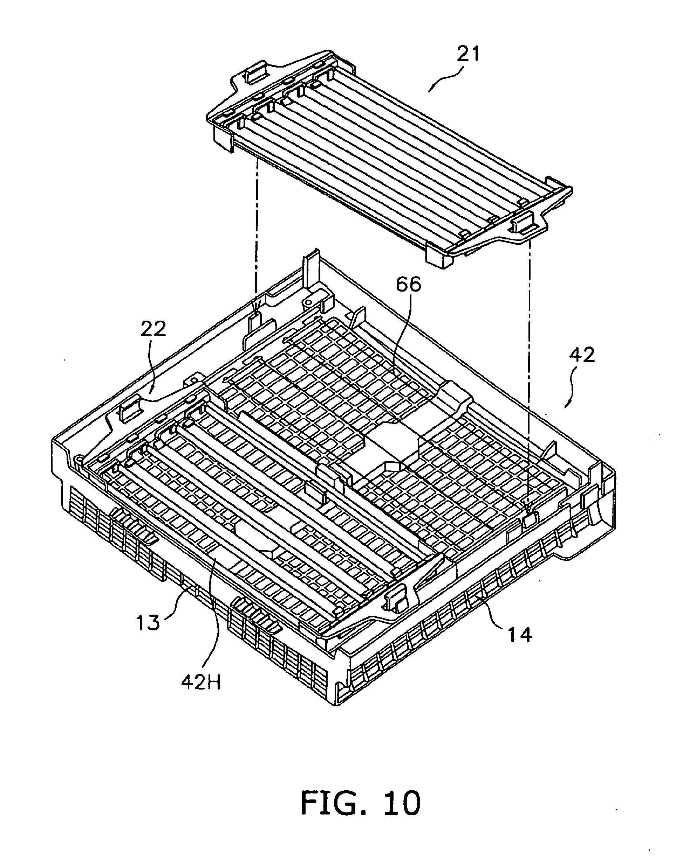

As shown in FIG. 2, the plasma ionization portion 42 is disposed in back of the prefilter 41 and is disposed on the back side of the second body portion 4. The plasma ionization portion 42 charges relatively small particles of dust floating in the air that pass through the prefilter 41. The plasma ionization portion 42 mainly includes, as shown in FIG. 10, a pair of opposing electrodes 21 and 22 and plural ionization wires 66 (wire electrode portions). Further, in FIG. 10, a reference numeral is added to only some of the ionization wires 66 of the plural ionization wires 66, and others are omitted.

### (Opposing Electrodes 21 and 22)

The pair of opposing electrodes 21 and 22 are, as shown in FIG 10, metal plates that have a square-wave shaped cross section and are vertically divided and attached. The opposing electrodes 21 and 22 are disposed in proximity to the ionization wires 66 and create a corona discharge between themselves and the ionization wires 66 as a result of a high voltage current being allowed to flow in the ionization wires 66.

### (Ionization Wires 66)

The plural ionization wires 66, together with the opposing electrodes 21 and 22, fulfill the role of charging relatively small particles of dust floating in the air that pass through the prefilter 41. The ionization wires 66 are disposed in front of the opposing electrodes 21 and 22. The discharge voltage is applied to these ionization wires 66, whereby a corona discharge arises between the ionization wires 66 and the opposing electrodes 21 and 22. The ionization wires 66 are disposed so as to intersect an air path and are disposed in an air flow that passes through the air path. Further, the ionization wires 66 are disposed in parallel in the vertical direction, and the plural ionization wires 66 are plurally arranged and disposed in the horizontal direction. These ionization wires 66 are formed by tungsten wires or the like with a minute diameter and are used as discharge electrodes for charging the dust and the like.

### (Top Surface Guide 42G and Front Surface Guide 42H)

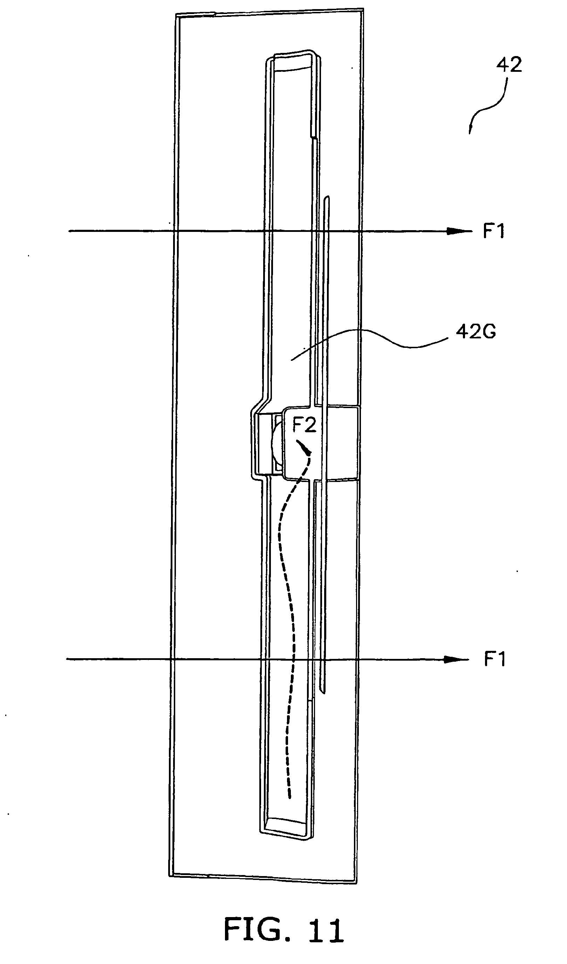

As shown in FIG. 11, in the top surface of the plasma ionization portion 42, there is formed the top surface guide 42G that is a groove that is long from side to side and is downwardly recessed. The top surface of this top surface guide 42G is covered by the first body portion 3 as a result of the plasma ionization portion 42 being attached to the first body portion 3 and configures part of the bypass air flow path. The right side of this top surface guide 42G is configured such that the bypass air flow F2 that includes the active species flows in from the open portion on the front side of the aforementioned unit housing portion B. Additionally, the bypass air flow F2 that has flowed in in this manner flows downward from the vicinity of the center of the top surface guide 42G.

Further, as shown in FIG 10, FIG 13 and FIG. 14, in the vicinity of the center of the front surface of the plasma ionization portion 42, there is disposed the front surface guide 42H that is configured by a groove that is disposed vertically and recessed rearward and a rib 43R that projects frontward. This front surface guide 42H has a shape that corresponds to the contour of the partition ventilation portion 41 b of the aforementioned prefilter 41. Additionally, as shown in FIG 12, the back surface of the prefilter 41 contacts the rib 43R of the plasma ionization portion 42, the bypass air flow path is formed between the partition ventilation portion 41 b of the prefilter 41 and the front surface guide 42H, and the bypass air flow from the top surface guide 42G leads downward.

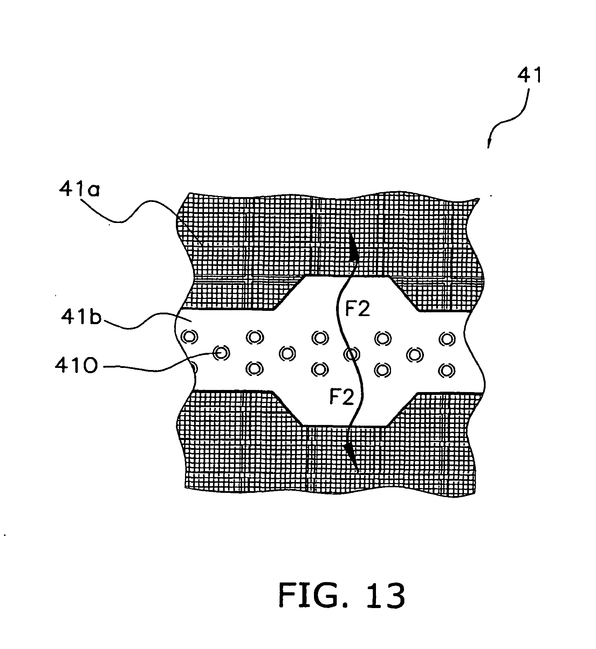

At this time, the bypass air flow F2 resulting from the air that includes the active species that passes through the front surface guide 42H is, as shown in FIG. 13, released toward the front of the filter portions 41a via the holes 410 in the partition ventilation portion 41b.

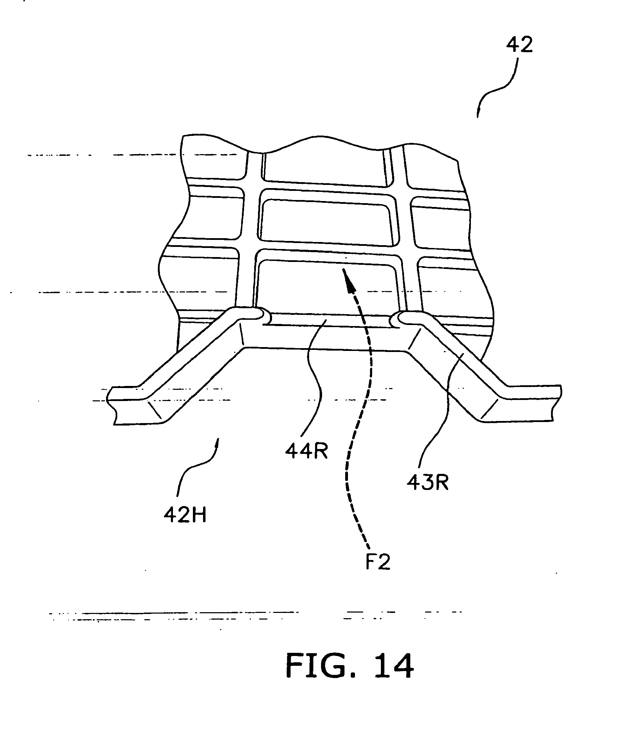

It will be noted that, as for the rib 43R that is disposed in the plasma ionization portion 42, as shown in FIG. 13 and FIG. 14, on the portion that corresponds to part of the bulging portions of the partition ventilation portion 41 b, there is disposed a recessed portion 44R that is backwardly recessed. Thus, part of the bypass air flow F2 that includes the active species is provided also to the downstream side of the prefilter 41 so that the active species can be sufficiently provided with respect also to the cleaning portion in back of the prefilter 41.

### {Air Cleaning Action Resulting from Air Cleaning Unit}

This air cleaner 1 is, as shown in FIG. 5, equipped with the air cleaning unit 40 (air cleaning portion), which comprises the prefilter 41, the plasma ionization portion 42, the photocatalytic filter 43 and the plasma catalytic filter 44, and the streamer discharge unit 63, and the air cleaner 1 removes foreign matter that is included in the air inside the room that has been sucked in from each of the suction openings (the lower suction opening 13 and the side suction openings 14) and cleans the air. Below, the air cleaning action resulting from the air cleaning unit 40 will be described.

The air inside the room that has been sucked in from the lower suction opening 13 and the side suction openings 14 first passes through the filter portions 41a of the prefilter 40. At this time, relatively large particles of dust are removed from the air. Further, because of the action of the photocatalyst and the catechin that are included in the prefilter 41, the breeding of viruses and fungi such as mold fungi and bacteria that are included in the dust and the like that adheres to the filter portions 41 a of the prefilter 41 is suppressed, and viruses are inactivated.

The air flow that has passed through the prefilter 41 passes between the ionization wires 66 and the opposing electrodes 21 and 22. When a high voltage is applied between the ionization wires 66 and the opposing electrodes 21 and 22, discharge arises between the ionization wires 66 and the opposing electrodes 21 and 22. As a result, dust and the like that is included in the air flow that passes between the ionization wires 66 and the opposing electrodes 21 and 22 charges to a positive charge.

The air flow that has passed between the ionization wires 66 and the opposing electrodes 21 and 22 passes through the photocatalytic filter 43. At this time, dust and the like that has been charged when the air passed through the plasma ionization portion 42 is adsorbed by the electrostatic filter. Further, dust and the like that has passed through the electrostatic filter is adsorbed by the titanium apatite-carrying filter. It will be noted that, because viruses, fungi and the like that are included in the dust are also charged when the air passes between the ionization wires 66 and the opposing electrodes 21 and 22, the adsorption efficiency of viruses and fungi to the titanium apatite rises.

The air flow that has passed through the photocatalytic filter 43 passes through the plasma catalytic filter 44. In the plasma catalytic filter 44, viruses, fungi and the like in the air that were not adsorbed by the photocatalytic filter 43 are adsorbed.

The main air flow F1 that has passed through the plasma catalytic filter 44 and has been cleaned is blown out into the inside of the room from the blowout opening 12. Further, some of the air that has passed through the plasma catalytic filter 44 is not blown out into the inside of the room but flows toward the bypass air flow path and becomes the bypass air flow F2.

In the bypass air flow path, as mentioned above, there is disposed the streamer discharge unit 63. In the streamer discharge unit 63, a direct current, an alternating current or a pulse discharge voltage is applied between the streamer discharge electrodes 70 and the ground plate 73, and a streamer discharge arises between the streamer discharge electrodes 70 and the ground plate 73. When the streamer discharge arises, low-temperature plasma is generated in the discharge field, and the active species are created inside the discharge unit casing 69 and are released into the bypass air flow F2. It will be noted that the energy level of these active species is extremely high and that these active species have the capability of dissolving and deodorizing small organic molecules that are included in the air, such as types of ammonia, types of aldehyde and nitrogen oxides, even before they reach the photocatalytic filter 43.

Here, the air that passes the vicinity of the streamer discharge electrodes 70 is cleaned in advance in the prefilter 41, the plasma ionization portion 42, the photocatalytic filter 43 and the plasma catalytic filter 44. For this reason, a situation where dust collects, ammonium nitrate or the like adheres to the terminal portions of the streamer discharge electrodes 70 can be prevented. Thus, the streamer discharge can be stably generated.

The bypass air flow F2 that includes the active species in this manner passes through the bypass air flow path, is provided to the front and back of the prefilter 41, and can act on and clean dust and the like adhering to the prefilter 41.

Additionally, the air that includes the active species passes between the ionization wires 66 and the opposing electrodes 21 and 22 and passes through the photocatalytic filter 43. At this time, dust and the like that has been charged when the air passes through the plasma ionization portion 42 is adsorbed by the electrostatic filter. Additionally, at the time the dust and the like that has passed through the electrostatic filter is adsorbed by the titanium apatite-carrying filter, the photocatalytic function of the titanium apatite is activated by the active species that are supplied from the streamer discharge unit 63 such that the titanium apatite can inactivate or kill viruses, mold fungi, bacteria and the like.

Moreover, in the plasma catalytic filter 44, viruses, fungi and the like in the air that were not adsorbed by the photocatalytic filter 43 are adsorbed, and these fungi, viruses and the like are killed or inactivated by the titanium dioxide that has been activated by the air that includes the active species.

### <Characteristics of Air Cleaner 1 of Present Embodiment>

In the air cleaner 1 of the present embodiment, the bypass air flow that passes through the streamer discharge unit 63 is cleaned in the main air flow path. For this reason, a situation where contaminants that are present in the air end up adhering to the discharge portion 83 can be prevented. Thus, plasma discharge can be stably performed. Thus, the active species can be stably provided with respect to the air cleaning portion 40, so the air cleaning effect can be stabilized.

### <Modifications of Air Cleaner of Present Embodiment>

### (A)

In the air cleaner 1 of the preceding embodiment, a case where the active species are supplied to the front and the back of the prefilter 41 has been cited as an example and described.

However, for example, the active species may also be supplied to just the back of the prefilter 41, and effects resulting from stable generation of the active species can be obtained even in back of the plasma ionization portion 42.

### (B)

Further, for example, a deodorizing filter that adsorbs the active species may also be disposed, and a bio-antibody filter may also be optionally disposed on the downstream side thereof. Thus, the deodorizing filter adsorbs the active species, whereby odorous components can be decomposed and a bio-antibody can be allowed to function while being put to use.

### (C)

In the air cleaner 1 of the preceding embodiment, a case where some of the air that is about to flow out from the vicinity of the blowout opening 12 becomes the bypass air flow F2, passes through the opening in the unit housing portion B and is supplied to the streamer discharge unit 63 has been cited as an example and described.

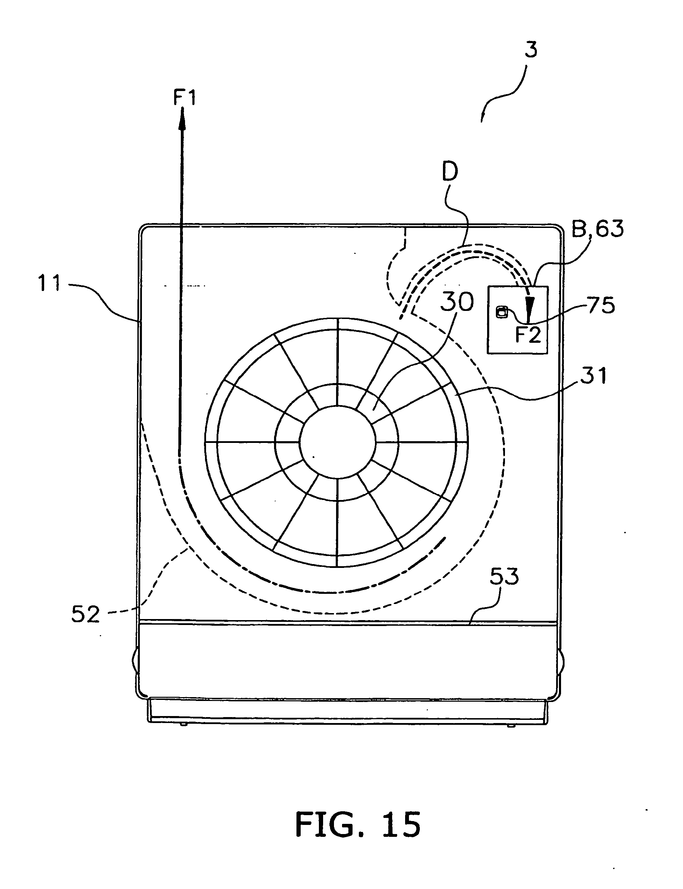

However, the for example, the air cleaner 1 may also be given a configuration which, as shown in FIG. 15, separately from the passage that leads from the blowing fan 31 to the blowout opening 12, employs an extension passage D that can supply the air flow from the blowing fan 31 directly to the unit housing portion B and the streamer discharge unit 63.

### (D)

In the air cleaner 1 of the preceding embodiment, a case where one blowing fan 31 is disposed on the downstream-most side in the vicinity of the blowout opening 12 has been cited as an example and described.

However, for example, the disposition of the blowing fan 31 may also be such that both the main air flow F 1 and the bypass air flow F2 of the preceding embodiment are formed. For example, the blowing fan 31 may also be disposed between any of the prefilter 41, the plasma ionization portion 42, the photocatalytic filter 43 and the plasma catalytic filter 44 that configure the air cleaning unit 40.

Further, the blowing fan 31 does not have to be one, and the air cleaner 1 may also be given a configuration that is disposed with two of the blowing fans: a blowing fan for mainly forming the main air flow F 1 and a blowing fan for mainly forming the bypass air flow F2. In this case, the blowing fan that generates the bypass air flow F2 may be disposed in the middle of the bypass air flow path so as to forcibly generate the bypass air flow F2.

### INDUSTRIAL APPLICABILITY

By utilizing the present invention, even when there is employed a cleaning function for which there is the potential for the exhibition efficiency of the function to be reduced by contaminants in the air, the exhibition of that function can be stabilized, so the present invention is particularly useful as an air cleaner that is equipped with plural cleaning functions.

## Claims

1. An air cleaner (1) that allows air of a target space to pass therethrough and cleans the air, the air cleaner comprising:
- a first flow path that interconnects a suction opening for sucking in the air of the target space and a blowout opening for blowing out the air with respect to the target space;
- a first cleaning portion (40) disposed in the first flow path and having a filter means (41 a) that passes therethrough from an upstream side to a downstream side;
- a second flow path that interconnects a downstream side connecting space between the first cleaning means and the blowout opening in the first flow path and a upstream side connecting space between the first cleaning means and the suction opening in the first flow path;
- an additional function of causing the air that passes therethrough from the downstream side of the first cleaning portion to the upstream side of the first cleaning portion in a state that additionally allows a second cleaning function that is different from the first cleaning function to be exhibited in the first cleaning portion (40), comprising a second cleaning means (63) disposed in the second flow path and having a wire electrode (70) and an opposing electrode (73),
**characterized in that**
- a blower (31) disposed in a position in the first flow path between the first cleaning means and the downstream side connecting space, and at which position the blower (31) makes both the vicinity of the blowout opening and the vicinity of one end of the second flow path facing to the downstream side connecting space into a positive pressure as a result of the blower (31) being driven to rotate; and connecting space into a positive pressure as a result of the blower (31) being driven to rotate; and
- the blower (31) forms an air flow that leads from the suction opening to the blowout opening in the first flow path and an air flow that leads from the downstream side connecting space to the upstream side connecting space in the second flow path.

2. The air cleaner (1) of claim 1, wherein the second cleaning means targets only at least some of the air that has been cleaned by the first cleaning function and allows the additional function to be exhibited.

3. The air cleaner (1) of claim 1 or 2, wherein an upstream side end portion of the second flow path at least includes a portion extending as far as the vicinity of a center of a surface that is perpendicular to a flow direction of the first flow path.

4. The air cleaner (1) of any one of claims 1 to 3, wherein
- the second cleaning means includes a wire electrode (21) and an opposing electrode (22) that is positioned in the vicinity of the wire electrode, and
- the first cleaning means includes an adsorbing means that adsorbs a cleaning target that passes therethrough and is activated by the plasma discharge.

5. The air cleaner (1) of claim 4, wherein the first cleaning means further includes an electrostatic filter disposed on the upstream side of the adsorbing means and an ionizing means disposed further on the upstream side of the electrostatic filter and charging the cleaning target oppositely from the electrostatic filter.

6. The air cleaner (1) of claim 5, wherein the first cleaning means further includes a net-like prefilter on the upstream side of the ionization means.

7. The air cleaner (1) of claim 1, wherein the wire electrode (70) is a plasma discharge electrode.

8. The air cleaner (1) of claim 1, wherein the wire electrode (70) is a streamer discharge electrode.

## Patentansprüche

1. Luftreiniger (1), der das Durchtreten von Luft eines Zielraums durch diesen erlaubt und die Luft reinigt, wobei der Luftreiniger aufweist:
- einen ersten Strömungsweg, der eine Ansaugöffnung zum Ansaugen der Luft aus dem Zielraum und eine Ausblasöffnung zum Ausblasen der Luft im Hinblick auf den Zielraum miteinander verbindet;
- einen ersten Reinigungsabschnitt (40), der im ersten Strömungsweg angeordnet ist und der ein Filtermittel (41) hat, das durch diesen von stromaufwärts nach stromabwärts durchtritt;
- einen zweiten Strömungsweg, der einen stromabwärts gelegenen Verbindungsraum zwischen dem ersten Reinigungsmittel und der Ausblasöffnung im ersten Strömungsweg und einen zweiten stromaufwärts gelegenen Verbindungsraum zwischen dem ersten Reinigungsmittel und der Ansaugöffnung im ersten Strömungsweg miteinander verbindet;
- eine zusätzliche Funktion, die veranlasst, dass die Luft, die durch diesen von stromabwärts des ersten Reinigungsabschnitts nach stromaufwärts des ersten Reinigungsabschnitts in einem Zustand durchtritt, der zusätzlich eine zweite Reinigungsfunktion ermöglicht, die von der ersten Reinigungsfunktion, die im ersten Reinigungsabschnitt (40) ausgeführt wird, verschieden ist, mit einem zweiten Reinigungsmittel (63), das im zweiten Strömungsweg angeordnet ist und eine Drahtelektrode (70) und eine Gegenelektrode (73) hat,
**dadurch gekennzeichnet, dass**
- ein Gebläse (31) in einer Position im ersten Strömungsweg zwischen dem ersten Reinigungsmittel und dem stromabwärts gelegenen Verbindungsraum angeordnet ist, und an welcher Position das Gebläse (31) sowohl die Umgebung der Ausblasöffnung als auch die Umgebung des einen Endes des zweiten Strömungswegs, der dem stromabwärts gelegenen Verbindungsraum zugewandt ist, unter Überdruck setzt als Ergebnis des zum Drehen angetriebenen Gebläses (31); und
- das Gebläse (31) einen Luftstrom bildet, der von der Ansaugöffnung zur Ausblasöffnung im ersten Strömungsweg führt, und einen Luftstrom, der vom stromabwärts gelegenen Verbindungsraum zum stromaufwärts gelegenen Verbindungsraum im zweiten Strömungsweg führt.

2. Luftreiniger (1) nach Anspruch 1, wobei das zweite Reinigungsmittel auf nur wenigstens einiges der Luft abzielt, die von der ersten Reinigungsfunktion gereinigt worden ist und ermöglicht, die zusätzliche Funktion auszuführen.

3. Luftreiniger (1) nach Anspruch 1 oder 2, wobei ein stromaufwärts gelegener Endabschnitt des zweiten Strömungswegs wenigstens einen Abschnitt aufweist, der sich so weit wie die Umgebung eines Zentrums einer Oberfläche erstreckt, die senkrecht zur Strömungsrichtung des ersten Strömungswegs ist.

4. Luftreiniger (1) nach einem der Ansprüche 1 bis 3, wobei
- das zweite Reinigungsmittel eine Drahtelektrode (21) aufweist und eine Gegenelektrode (22), die in der Umgebung der Drahtelektrode angeordnet ist, und
- das erste Reinigungsmittel ein adsorbierendes Mittel aufweist, das ein Reinigungszielobjekt adsorbiert, das durch dieses durchtritt und durch die Plasmaentladung aktiviert wird.

5. Luftreiniger (1) nach Anspruch 4, wobei das erste Reinigungsmittel weiter einen elektrostatischen Filter, der stromaufwärts des adsorbierenden Mittels angeordnet ist, und ein Ionisierungsmittel aufweist, das weiter stromaufwärts des elektrostatischen
Filters angeordnet ist und das Reinigungszielobjekt gegenüber vom elektrostatischen Filter auflädt.

6. Luftreiniger (1) nach Anspruch 5, wobei das erste Reinigungsmittel weiter einen netzähnlichen Vorfilter stromaufwärts des Ionisierungsmittels aufweist.

7. Luftreiniger (1) nach Anspruch 1, wobei die Drahtelektrode (70) eine Plasma-Entladungselektrode ist.

8. Luftreiniger (1) nach Anspruch 1, wobei die Drahtelektrode (70) eine "Streamer"-Entladungselektrode ist.

## Revendications

1. Épurateur d'air (1) qui permet à l'air d'un espace cible de passer au travers de celui-ci et qui épure l'air, l'épurateur d'air comportant :
- un premier circuit d'écoulement qui raccorde une ouverture d'aspiration destinée à aspirer l'air de l'espace cible et une ouverture de purge destinée à purger l'air par rapport à l'espace cible ;
- une première partie de nettoyage (40) disposée dans le premier circuit d'écoulement et ayant un moyen de filtration (41a) qui traverse celle-ci depuis un côté amont jusqu'à un côté aval ;
- un second circuit d'écoulement qui raccorde un espace de raccordement côté aval entre le premier moyen de nettoyage et l'ouverture de purge dans le premier circuit d'écoulement et un espace de raccordement côté amont entre le premier moyen de nettoyage et l'ouverture d'aspiration dans le premier circuit d'écoulement ;
- une fonction supplémentaire consistant à entraîner l'air qui passe au travers de celui-ci depuis le côté aval de la première partie de nettoyage jusqu'au côté amont de la première partie de nettoyage dans un état qui permet par ailleurs à une seconde fonction de nettoyage qui est différente de la première fonction de nettoyage d'être exploitée dans la première partie de nettoyage (40), comportant un second moyen de nettoyage (63) disposé dans le second circuit d'écoulement et ayant une électrode en fil (70) et une électrode opposée (73), **caractérisé en ce que**
- un souffleur (31) disposé en une position dans le premier circuit d'écoulement entre le premier moyen de nettoyage et l'espace de raccordement côté aval, et une position sur laquelle le souffleur (31) transforme à la fois la proximité de l'ouverture de purge et la proximité d'une extrémité du second circuit d'écoulement orienté vers l'espace de raccordement côté aval en une pression positive du fait que le souffleur (31) est entraîné pour tourner ; et
- le souffleur (31) forme un écoulement d'air qui mène de l'ouverture d'aspiration jusqu'à l'ouverture de purge dans le premier circuit d'écoulement et un écoulement d'air qui mène de l'espace de raccordement côté aval jusqu'à l'espace de raccordement côté amont dans le second circuit d'écoulement.

2. Épurateur d'air (1) selon la revendication 1, dans lequel le second moyen de nettoyage cible uniquement au moins une partie de l'air qui a été épuré par la première fonction de nettoyage et permet à la fonction supplémentaire d'être exploitée.

3. Épurateur d'air (1) selon la revendication 1 ou la revendication 2, dans lequel une partie d'extrémité côté amont du second circuit d'écoulement comprend au moins une partie s'étendant jusqu'à la proximité d'un centre d'une surface qui est perpendiculaire à la direction de l'écoulement du premier circuit d'écoulement.

4. Épurateur d'air (1) selon l'une quelconque des revendications 1 à 3, dans lequel
- le second moyen de nettoyage comprend une électrode en fil (21) et une électrode opposée (22) qui est positionnée à proximité de l'électrode en fil, et
- le premier moyen de nettoyage comprend un moyen d'adsorption qui adsorbe une cible de nettoyage qui passe au travers de celui-ci et qui est activé par la décharge de plasma.

5. Épurateur d'air (1) selon la revendication 4, dans lequel le premier moyen de nettoyage comprend par ailleurs un filtre électrostatique disposé du côté amont du moyen d'adsorption et un moyen de ionisation disposé plus loin du côté amont du filtre électrostatique et chargeant la cible de nettoyage de manière opposée par rapport au filtre électrostatique.

6. Épurateur d'air (1) selon la revendication 5, dans lequel le premier moyen de nettoyage comprend par ailleurs un préfiltre du type à filet du côté amont du moyen de ionisation.

7. Épurateur d'air (1) selon la revendication 1, dans lequel l'électrode en fil (70) est une électrode de décharge à plasma.

8. Épurateur d'air (1) selon la revendication 1, dans lequel l'électrode en fil (70) est une électrode de décharge à sillages lumineux.
